# EUROPÄISCHE PATENTSCHRIFT

(11) **EP 3 926 202 B1**
(45) Veröffentlichungstag und Bekanntmachung des Hinweises auf die Patenterteilung: **25.10.2023**
(21) Anmeldenummer: 21190564.1
(22) Anmeldetag: 17.09.2018
(51) Int. Cl.: F16F 3/02, A61F 2/50

(54) **FEDEREINRICHTUNG UND HYDRAULIKAKTUATOR**
SPRING MECHANISM AND HYDRAULIC ACTUATOR
DISPOSITIF Á RESSORT ET ACTIONNEUR HYDRAULIQUE

(30) Priorität: 10.11.2017 DE 102017126396
(43) Veröffentlichungstag der Anmeldung: 22.12.2021
(62) Teilanmeldung aus: 18779573.7
(73) Patentinhaber: Otto Bock Healthcare Products GmbH, 1110 Wien (AT)
(72) Erfinder: HARTL, Stefan, 1160 Wien (AT)
(74) Vertreter: Gramm, Lins & Partner Patent- und Rechtsanwälte PartGmbB

(56) Entgegenhaltungen:
- DE-A1-102006 009 510
- DE-A1-102012 108 322
- DE-B- 1 024 769

## Beschreibung

Die Erfindung betrifft eine Federeinrichtung mit zumindest zwei Wellenscheiben und zumindest einer zwischen den Wellenscheiben angeordneten Federscheibe sowie einen Hydraulikaktuator mit einer solchen Federeinrichtung.

Energiespeicher zur Speicherung mechanischer Energie können vielfältig ausgebildet sein. Neben hydraulischen und pneumatischen Energiespeichern gibt es mechanische Energiespeicher, bei denen Energie über eine elastische Verformung gespeichert wird. Zur Abgabe dieser Energie erfolgt eine Rückverformung, es handelt sich somit um eine elastische Verformung oder eine reversible Deformation. Solche Energiespeicher sind mechanische Federn, die auf vielfältige Arten und Weisen aufgebaut sein können. Neben Elastomerelementen können Kunststofffedern, Federn aus faserverstärkten Kunststoffen oder Metallfedern zur mechanischen Energiespeicherung eingesetzt werden. Häufige Bauformen solcher Federn sind Blattfedern, Spiralfedern oder auch Wendel- oder Schraubenfedern.

Die Federvorspannung kann insbesondere bei Schraubenfedern durch Veränderung der Ausgangsfederlänge eingestellt werden.

Insbesondere bei orthopädietechnischen Einrichtungen wie Orthesen oder Prothesen mit einem System zu einer Energierückgewinnung während einer Bewegung besteht das Problem, dass nur ein begrenzter Bauraum zur Verfügung steht und eine Speicherfeder hinsichtlich der aufzunehmenden Energiemenge beziehungsweise der abzugebenden Energiemenge bei Energierückgewinnungssystem schwierig an die jeweiligen Betriebszustände und Anforderungen angepasst werden kann. Daher werden häufig nicht einstellbare Speicherfedern eingesetzt, die für den jeweiligen Einsatzzweck und den jeweiligen Patienten angepasst ausgewählt werden müssen.

Die DE 10 2012 108 322 A1 offenbart ein System zum Verringern von Geräuschen einer Bypassventilvorrichtung mit einem Bypassventil, einem Aktuator und einer Verbindungseinheit. Die Verbindungseinheit weist mehrere Scheiben auf, wobei eine Scheibe eine Mehrzahl von Stützplatten und eine Wellenplatte aufweist. Die Wellenplatte ist zwischen den Stützplatten angeordnet und mit wenigstens einer Welle ausgebildet. Wenn eine Vibration auftrifft, verringert die Wellenplatte die Vibration, so dass das Geräusch verringert wird.

Die DE 1 024 769 B betrifft eine Einrichtung zur Umwandlung von Drehbewegungen in Hubbewegungen mit einer Drehachse, auf der abwechselnd unverdrehbare gewellte Scheiben und verdrehbare gewellte Scheiben angeordnet sind. Die Scheiben werden mit einer Feder zusammengehalten.

Die DE 10 2006 009 510 A1 offenbart eine Hüftgelenkprothese mit einem Anschlussmittel zur Befestigung an einer Befestigungseinrichtung und an einem Kunstbein. An der Hüftgelenkprothese ist eine Energiespeichereinheit in Form eines Federelementes vorgesehen, die bei einer Streckung des Kunstbeines im Hüftgelenk Energie speichert und diese zur Unterstützung der Beugebewegung an das Kunstbein abgibt.

Aufgabe der vorliegenden Erfindung ist es daher eine Federeinrichtung und einen Hydraulikaktuator mit einer solchen Federeinrichtung bereitzustellen, die ein geringes Bauvolumen benötigt und an den jeweiligen Einsatzzweck einstellbar ist. Als mögliche unterschiedliche Einsatzzwecke sind insbesondere die Individualisierung im Hinblick auf den Patienten sowie die Anpassung an sich ändernde Betriebszustände von Maschinen, so dass im Bereich der Regelungstechnik die notwendigen oder gewünschten Anpassungen vorgenommen werden können.

Erfindungsgemäß wird diese Aufgabe durch eine Federeinrichtung mit den Merkmalen des Hauptanspruches und einen Hydraulikaktuator mit den Merkmalen des nebengeordneten Anspruchs gelöst. Vorteilhafte Ausgestaltungen und Weiterbildungen der Erfindung sind in den Unteransprüchen, der Beschreibung sowie den Figuren offenbart.

Die erfindungsgemäße Federeinrichtung mit zumindest zwei Wellenscheiben aus einem formstabilen Material, die starr ausgeführt sind und zumindest eine gewellte Oberfläche aufweisen, sieht vor, dass zwischen zwei Wellenscheiben zumindest eine Federscheibe angeordnet ist, wobei die Wellenscheiben zueinander verdrehbar gelagert sind und zumindest eine Wellenscheibe mit einer motorisch angetriebenen Verstelleinrichtung gekoppelt ist. Durch die verdrehbare Anordnung der beiden Wellenscheiben, zwischen denen die zumindest eine Federscheibe angeordnet ist, kann eine stufenlose Anpassung an den jeweiligen Einsatzzweck erfolgen. Durch das Verdrehen der einen Wellenscheibe relativ zu der anderen Wellenscheibe kann der Hub der Federeinrichtung eingestellt werden. Finden sich die jeweiligen Maxima und Erhebungen der einander gegenüberliegenden Wellenscheiben in einer Position, in der sie unmittelbar einander gegenüberliegen, befindet sich die Federscheibe direkt zwischen den beiden Maxima, wobei jedoch keine axiale Relativbewegung der beiden Wellenscheiben aufeinander zu möglich ist. Dementsprechend kann die Federscheibe, die bevorzugt als ebene, insbesondere metallische Federscheibe ausgebildet ist, nicht elastisch verformt werden. Vielmehr findet nur eine zu vernachlässigende Materialstauchung in dem Bereich der einander gegenüberliegenden Maxima statt. Der effektive Federweg der Federeinheit ist somit gleich Null. Werden die beiden Federscheiben zueinander so verdreht, dass ein Maxima einem Minima gegenüberliegt, also eine Erhebung einer Vertiefung, ist eine Maximalverlagerung in Axialrichtung, also in Richtung der Wellenscheiben aufeinander zu möglich. Dadurch ist eine maximale Verformung der zwischen diesen Wellenscheiben angeordneten Federscheibe oder Federscheiben möglich, wodurch eine maximale Energiemenge gespeichert werden kann. Durch die Verdrehbarkeit der beiden Wellenscheiben zueinander ist eine stufenlose Verstellung der Federeinrichtung zwischen diesen Grenzeinstellungen hinsichtlich der Steifigkeit und Vorspannung durch eine mechanische Federeinrichtung gegeben. Es wird nur ein geringes Bauvolumen, insbesondere in einer Axialerstreckung, benötigt und darüber hinaus wird bei einer Verwendung dieser Federeinrichtung in einem hydraulischen System, insbesondere in einem Hydraulikaktuator wie einem Hydraulikdämpfer oder einer hydraulischen Betätigungseinrichtung, kein zusätzliches Ausgleichsvolumen benötigt. Vorteilhafterweise ist die Verstelleinrichtung motorisch angetrieben, insbesondere elektromotorisch, um bei orthopädietechnischen Einrichtungen, beispielsweise Orthesen oder Prothesen, während der Benutzung eine Anpassung des Federweges, der Steifigkeit und der aufzunehmenden oder abzugebenden Energiemenge vornehmen zu können. Der Motor ist über eine Steuereinrichtung in die ein oder andere Richtung antreibbar, sodass vorteilhafterweise mit nur einem Motor sowohl eine Vergrößerung als auch eine Verringerung der Federwegslänge und der aufzunehmenden Energie erfolgen kann. Mit einem Motor können auch mehrere Verstelleinrichtungen angetrieben werden und beispielsweise über ein Getriebe eine gegenläufige Verstellbewegung zweier Wellenscheiben bewirkt werden. Die Steuereinrichtung kann mit Sensoren verbunden sein, die an der orthopädietechnischen Einrichtung angeordnet sind, sodass auf unterschiedliche Anforderungen während der Benutzung der Federeinrichtung reagiert werden kann. Die von den Sensoren ermittelten Werte werden in der Steuereinrichtung ausgewertet, mit dort hinterlegten Programmen oder Kriterien verglichen und weiterverarbeitet zu Stellsignalen, mit denen der Motor versorgt wird, um eine Verstellung der Position der Wellenscheiben zueinander in die eine oder andere Richtung vorzunehmen.

Eine Weiterbildung der Erfindung sieht vor, dass mehrere Federscheiben als ein Federscheibenstapel zwischen zwei Wellenscheiben angeordnet sind. Durch die Hintereinanderschaltung mehrerer Federscheiben und die Zusammenfassung mehrerer Federscheiben zu einem Federscheibenstapel kann durch Hinzufügen oder Entfernen einer Federscheibe eine einfache Anpassung an die gewünschte Federsteifigkeit oder an die gewünschte zu speichernde und abzugebende Energiemenge erreicht werden. Beispielsweise kann dadurch eine Anpassung an Patienten, die ein orthopädietechnisches System mit einem Hydraulikaktuator verwenden, an unterschiedliche Aktivitätsniveaus oder an unterschiedliche Gewichtsklassen erfolgen.

In einer Weiterbildung der Erfindung ist es vorgesehen, dass eine endseitig angeordnete Wellenscheibe auf der der Federscheibe abgewandten Seite eben ausgebildet ist. Wellenscheiben können somit bevorzugt eine Wellenform aufweisen, die in der Seitenansicht einen sinusartigen Verlauf auf der Oberseite und auf der Unterseite aufweisen. Um ein möglichst geringes Bauvolumen realisieren zu können und den Federdruck gleichmäßig auf ein Widerlager ableiten zu können, ist bei einer endseitig angeordneten Wellenscheibe nur diejenige Oberfläche gewellt ausgebildet, die der Federscheibe zugeordnet ist. Die Rückseite ist eben ausgebildet, sodass eine vollflächige und nicht nur linienartige Anlage in den Bereichen der Erhebungen, die auf der gegenüberliegenden Seite als Vertiefungen ausgebildet sind, vorhanden ist. Bei nur zwei Wellenscheiben und einer dazwischen angeordneten Federscheibe oder einem dazwischen angeordneten Federscheibenstapel sind beide Wellenscheiben aufeinander abgewandten Seiten eben ausgebildet.

Bei einer Ausführungsform mit mehr als zwei Wellenscheiben ist es bevorzugt vorgesehen, dass zwischen zwei Federscheiben oder zwei Federscheibenstapeln eine Wellenscheibe angeordnet ist, die auf beiden den Federscheiben zugewandten Seite wellig ausgebildet ist. Durch eine Hintereinanderschaltung mehrerer Wellenscheiben mit dazwischen angeordneten Federscheiben ist es möglich, sowohl den maximalen Hub als auch die maximal zu speichernde Energiemenge einzustellen. Je mehr Wellenscheiben hintereinander angeordnet sind, desto größer sind der maximale Hub und die maximal zu speichernde Energiemenge. Die zwischen zwei endseitig angeordneten Wellenscheiben angeordneten Wellenscheiben sind bevorzugt wellenförmig ausgebildet, sodass die jeweiligen Maxima in einer Ebene liegen. Die Ebenen, in denen die jeweiligen Maxima befindlich sind, sind bevorzugt parallel zueinander ausgebildet, das heißt, dass die Maxima auf der Vorderseite und die Maxima auf der Rückseite einer Wellenscheibe jeweils in einer Ebene liegen, wobei diese Ebenen bevorzugt parallel zueinander orientiert sind. Sind alle Maxima oder Erhebungen der Wellenscheiben so zueinander ausgerichtet, dass bei benachbarten Wellenscheiben die Maxima einander unmittelbar gegenüber liegen, ist die Maximallänge der Federeinrichtung erreicht, eine Federwirkung kann jedoch nicht erzielt werden, da keine Relativverlagerung der Wellenscheiben zueinander in Axialrichtung erfolgen kann. Sind die Wellenscheiben so zueinander ausgerichtet, dass die Maxima und Minima benachbarter Wellenscheiben zueinander ausgerichtet sind, kann eine maximale Verlagerung in Axialrichtung erfolgen und somit eine maximale Energiemenge gespeichert und abgegeben werden.

Die Wellenscheiben können durch Urformen, materialabtragende Bearbeitung einer ebenen Federscheibe oder durch Umformen hergestellt werden. Die Wellenscheiben sind aus einem soliden, starren Material hergestellt, beispielsweise Stahl oder einem anderen, formstabilen Metall oder beispielsweise einem faserverstärkten Kunststoff.

Die Wellenscheiben und die zumindest eine Federscheibe oder alle Federscheiben weisen in einer Variante jeweils eine zentrale Ausnehmung auf und sind in einer Weiterbildung der Erfindung auf einer Zentralführung gelagert. Sowohl die Federscheibe oder Federscheiben als auch die Wellenscheiben sind somit als Ringscheiben ausgebildet. Durch die Anordnung auf einer Zentralführung ist es möglich, eine Axialverlagerung der Wellenscheiben aufeinander zu oder voneinander weg zu führen, so dass eine lineare Bewegung sicher ausgeführt werden kann, ohne dass eine seitliche Ausweichbewegung stattfinden kann. Die Zentralausnehmung ist bevorzugt rund oder lässt zumindest eine Verdrehung der Wellenscheiben zueinander zu. Dazu können die Zentralausnehmungen abweichend von einer runden Ausgestaltung ausgebildet sein, beispielsweise mit einem Formschlusselement oder eine Abflachung oder einem Vorsprung, um eine Verdrehsicherung der Wellenscheiben oder zumindest einer Wellenscheibe relativ zu der Zentralführung zu gewährleisten, wenn dies notwendig oder gewünscht ist.

Zumindest eine Wellenscheibe ist, vorzugsweise über ein Formschlusselement, mit einer Verstelleinrichtung gekoppelt, um eine Verdrehung der Wellenscheiben zueinander zu ermöglichen. Das Formschlusselement kann als Vorsprung oder Ausnehmung, insbesondere als ein Sicherungsstift oder eine Bohrung zur Aufnahme eine Stiftes oder eine Eingriffselementes ausgebildet sein, ebenso ist es möglich, dass als Formschlusselement eine Innenkontur oder Außenkontur wirkt, die nicht rotationssymmetrisch ausgebildet ist. Beispielsweise kann die Außenkontur der Wellenscheibe eine Abflachung aufweisen oder eine polygonale Form haben, wobei die Verstelleinrichtung eine korrespondierende Abflachung, Kontur oder einen entsprechenden Vorsprung aufweist, sodass bei einer Verdrehung der Verstelleinrichtung um eine Drehachse die Wellenscheibe mitgenommen wird und sich relativ zu einer zweiten Wellenscheiben, die drehfest gelagert ist oder in eine andere Richtung verdreht wird, um die Drehachse bewegt wird. Bei einer Lagerung um eine Zentralführung kann das Formschlusselement oder die formschlüssige Kopplung der Verstelleinrichtung mit der Wellenscheibe über die zentrale Ausnehmung erfolgen, beispielsweise über eine unrunde Ausgestaltung der Zentralführung mit einer korrespondierenden Formgebung der zentralen Ausnehmung. Für den Fall einer endseitigen oder stirnseitigen Anordnung der Wellenscheiben ist es möglich, über eine Formschlussverbindung die an den der jeweiligen Federscheibe abgewandten Stirnfläche, insbesondere der äußeren Stirnfläche der Wellenscheibe, angreift, sodass eine Verdrehung relativ zu der jeweils anderen Wellenscheibe möglich ist. Das Formschlusselement oder die Formschlusselemente können als Ausnehmung, Vorsprung, Stift, Absatz, Unrundheit oder Verzahnung ausgebildet sein. Grundsätzlich besteht die Möglichkeit, dass bei einer beidseitig welligen Formgebung der Wellenscheibe über eine korrespondierende Welligkeit der Verstelleinrichtung eine formschlüssige Kopplung von Wellenscheibe und Verstelleinrichtung erfolgt. Alternativ zu einer Festlegung über ein Formschlusselement oder mehrere Formschlusselemente kann zumindest eine Wellenscheibe kraftschlüssig oder stoffschlüssig, z.B. durch Klemmen oder Kleben mit der Verstelleinrichtung gekoppelt werden. Auch eine Verstellung über ein Kraftübertragungselement ist möglich und vorgesehen.

Die Verstelleinrichtung kann die Wellenscheibe zumindest teilweise umgeben oder zumindest teilweise innerhalb der Verstelleinrichtung angeordnet sein. Beispielsweise kann die Verstelleinrichtung eine Hülse aufweisen, die außerhalb der Wellenscheibe angeordnet ist und diese umgibt, sodass neben einer Verdrehung gleichzeitig eine Führung in Axialrichtung durch die Verstelleinrichtung bereitgestellt wird. Alternativ oder ergänzend zu einer äußeren, umfänglich an der Wellenscheibe angeordneten Verstelleinrichtung kann diese auch innerhalb der Wellenscheibe in der jeweiligen Ausnehmung angeordnet sein, um dort eine Verdrehung um eine Längsachse oder Drehachse zu ermöglichen. Es besteht auch die Möglichkeit, dass mehrere Verstelleinrichtungen vorhanden sind, beispielsweise eine äußere, die Wellenscheibe umgebende Verstelleinrichtung, und eine innere, in einer Zentralausnehmung angeordnete Verstelleinrichtung, die gegenläufig agieren können, sodass zwei Wellenscheiben zueinander verdreht werden, indem beide Wellenscheiben in gegenläufige Richtungen verdreht werden.

Vorteilhafterweise ist die Verstelleinrichtung motorisch angetrieben, insbesondere elektromotorisch, um bei orthopädietechnischen Einrichtung, beispielsweise Orthesen oder Prothesen, während der Benutzung eine Anpassung des Federweges, der Steifigkeit und der aufzunehmenden oder abzugebenden Energiemenge vornehmen zu können. Der Motor ist über eine Steuereinrichtung in die ein oder andere Richtung antreibbar, sodass vorteilhafterweise mit nur einem Motor sowohl eine Vergrößerung als auch eine Verringerung der Federwegslänge und der aufzunehmenden Energie erfolgen kann. Mit einem Motor können auch mehrere Verstelleinrichtungen angetrieben werden und beispielsweise über ein Getriebe eine gegenläufige Verstellbewegung zweier Wellenscheiben bewirkt werden. Die Steuereinrichtung kann mit Sensoren verbunden sein, die an der orthopädietechnischen Einrichtung angeordnet sind, sodass auf unterschiedliche Anforderungen während der Benutzung der Federeinrichtung reagiert werden kann. Die von den Sensoren ermittelten Werte werden in der Steuereinrichtung ausgewertet, mit dort hinterlegten Programmen oder Kriterien verglichen und weiterverarbeitet zu Stellsignalen, mit denen der Motor versorgt wird, um eine Verstellung der Position der Wellenscheiben zueinander in die eine oder andere Richtung vorzunehmen.

Die Verstelleinrichtung kann eine Verzahnung aufweisen, die mit einem angetriebenen Zahnrad oder einer angetriebenen Schnecke gekoppelt ist, wodurch eine sehr feine Einstellung möglich ist. Darüber hinaus ist ein solcher Antrieb erprobt und kann über ein Getriebe leicht an die jeweiligen Anforderungen angepasst werden.

In einer Weiterbildung ist vorgesehen, dass alle Wellenscheiben die gleiche Anzahl an Wellen und die gleiche Wellenform aufweisen, um einerseits eine harmonische Verstellung und andererseits eine kostengünstige Herstellung von Wellenscheiben zu ermöglichen. Neben einer Ausgestaltung der Wellenform als eine Sinusform oder Abwandlungen davon, ist auch eine Dreiecksform, eine Trapezform oder eine Rechteckform eine Wellenform gemäß der vorliegenden Erfindung.

Die Federeinrichtung ist bevorzugt zwischen zwei zueinander verlagerbaren Endstücken angeordnet, wobei die Endstücke aufeinander zu verlagerbar sind, um elastisch die Federeinrichtung vorzuspannen, beziehungsweise die Verlagerung gegen eine Federkraft, die durch die Federeinrichtung aufgebracht wird, bewirken zu können.

Zumindest eine Wellenscheibe kann an oder in dem Endstück gelagert sein, insbesondere drehfest gelagert, sodass nur eine weitere Wellenscheibe verdreht werden muss, um den Federweg und die Federsteifigkeit einstellen zu können. An den Endstücken oder zumindest einem Endstück können Anschlusseinrichtungen oder Einrichtungen zum Festlegen der Federeinrichtung an anderen Komponenten ausgebildet oder angeordnet sein, sodass die Federeinrichtung als fertig montierbares Bauteil ausgeliefert werden kann. Das Endstück oder die Endstücke sind drehfest hinsichtlich der Drehbewegung der Wellenscheibe oder Wellenscheiben an der jeweiligen Komponente festlegbar. Ebenfalls kann ein Endstück als Kolben ausgebildet sein, der in einer Hydraulikeinheit mit einem Hydraulikfluid beaufschlagbar ist.

Zumindest eine Wellenscheibe kann drehfest an der Zentralführung und/oder an einem der Endstücke gelagert sein. Die Endstücke sind bevorzugt teleskopierbar aneinander festgelegt, sodass eine Relativbewegung in Axialrichtung aufeinander zu möglich ist. Die Verlagerungsrichtung entspricht im Wesentlichen der Längserstreckung der Drehachse der Wellenscheiben.

Die Endstücke können bevorzugt über die Zentralführung miteinander verbunden sein, wobei die Zentralführung gleichzeitig einen Begrenzungsanschlag ausbildet, sodass die beiden Endstücke nicht voneinander getrennt werden können. Über die Länge der Zentralführung ist es möglich, die Federvorspannung einzustellen oder die Maximalerstreckung der Federeinrichtung zu verändern. Dazu ist die Zentralführung längenveränderlich ausgebildet, beispielsweise über ein Gewinde.

Zwischen einem Endstück und zumindest einer Wellenscheibe kann ein weiteres Federelement angeordnet sein, insbesondere eine Schraubenfeder oder eine Wendelfeder, um einen vergleichsweise großen Federweg mit einer großen Energiespeicherfähigkeit bereitzustellen. Alternativ zu einem Federelement aus einer Wendelfeder oder Schraubenfeder kann dieses aus einem Elastomerwerkstoff hergestellt sein, das zwischen einem Endstück und einer stirnseitigen oder endseitigen Wellenscheibe angeordnet ist.

Bevorzugt weisen die Wellenscheiben eine sinusartige Form mit zumindest zwei Maxima und zwei Minima auf, wobei die sinusartige Form an zumindest einer Seite ausgebildet ist, die einer Federscheibe oder einem Federscheibenstapel gegenüberliegt. Wenn genau zwei Maxima und Minima vorhanden sind, ist eine Verkippsicherheit durch eine symmetrische Ausgestaltung gegeben. Ebenso sind dann maximale Federwege verwirklichbar.

Die Erfindung betrifft ebenfalls einen Hydraulikaktuator mit einer wie oben beschrieben Federeinrichtung. Über eine Kombination eines Hydraulikaktuators mit einer solchen Federeinrichtung ist es möglich, eine variable Energiespeicherung und Energieabgabe in ein hydraulisches System durchzuführen. Das hydraulische System ist insbesondere Teil einer orthopädietechnischen Einrichtung wie Orthese oder Prothese. Mit einer solchen Ausgestaltung ist es möglich, bei einer leichten Skalierbarkeit eine koaxiale Lösung eines Linearaktuators mit einer Federeinrichtung zu gewährleisten. Die Federeinrichtung mit einstellbarer Vorspannung ist in den Hydraulikaktuator integrierbar und es wird kein zusätzliches Ausgleichsvolumen durch den Einsatz einer Federeinrichtung mit zwei Wellenscheiben und zumindest einer dazwischen angeordneten Federscheibe benötigt.

Nachfolgend wird ein Ausführungsbeispiel der Erfindung anhand der beigefügten Figuren näher erläutert. Es zeigen:
- Figur 1-: eine Einzeldarstellung einer Federeinrichtung mit einem zusätzlichem Federelement in der Starrstellung;
- Figur 2-: eine Anordnung gemäß Figur 1 in Freigabestellung;
- Figur 3-: eine Anordnung gemäß Figur 1 in einer Zwischenstellung;
- Figur 4-: eine Federeinrichtung mit Verstelleinrichtung in perspektivischer Ansicht;
- Figur 5 -: eine Schnittdarstellung der Figur 4;
- Figur 6 -: eine Explosionsdarstellung einer Federeinrichtung;
- Figur 7 -: eine Darstellung gemäß Figur 6 mit Federelement;
- Figur 8 -: eine Schnittdarstellung durch einen Hydraulikaktuator mit montierter Federeinrichtung;
- Figur 9 -: Anwendungsbeispiele der Federeinrichtung in einer Orthese; sowie
- Figur 10 -: Anwendungsbeispiele einer Federeinrichtung in einer Prothese; sowie
- Figur 11 -: eine Darstellung des Federweges über der Federkraft.

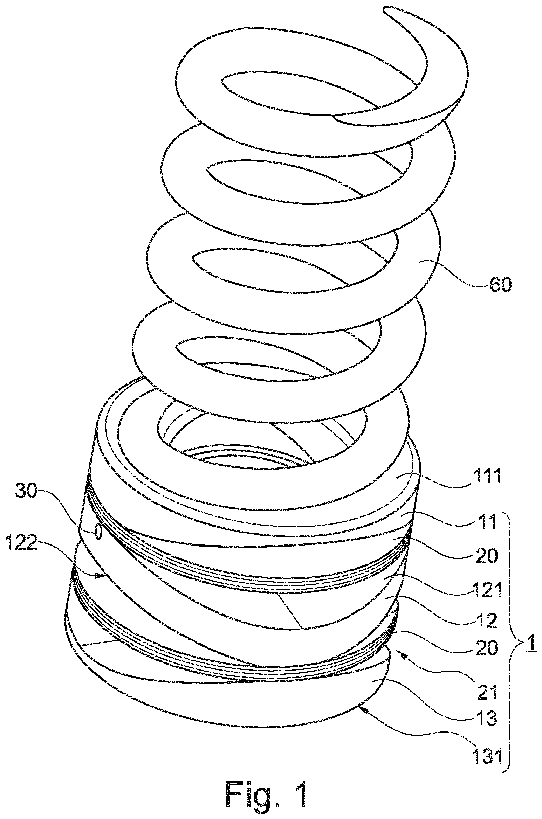

Figur 1 zeigt in einer Einzeldarstellung eine Federeinrichtung 1 in Kombination mit einem in Reihe des angeordneten Federelements 60. Die Federeinrichtung 1 besteht in dem dargestellten Ausführungsbeispiel aus zwei endseitigen Wellenscheiben 11, 13 und einer mittig dazwischen angeordneten Wellenscheibe 12. Alle Wellenscheiben 11, 12, 13 bestehen aus einem formstabilen Material und sind starr ausgeführt, so dass keine oder keine wesentlichen Verformungen innerhalb der Wellenscheiben 11, 12 ,13 bei einer Axialbelastung auftreten. Zwischen zwei Wellenscheiben 11, 12; 12, 13 befinden sich jeweils drei Federscheiben 20, die in dem unbelasteten Zustand eben ausgebildet sind. Die jeweils drei Federscheiben 20 bilden einen Federscheibenstapel 21 aus. Es können unterschiedliche Anzahlen an Federscheiben 20 zwischen zwei Wellenscheiben 11, 12, 13 angeordnet sein, um die zu speichernde Energiemenge oder die Federsteifigkeit zu verändern. Die beiden endseitigen Wellenscheiben 11, 13 weisen eine ebene Rückseite 111, 131 auf, die nicht mit einer Wellenkontur versehen ist. Auf der der ebenen Seite 111, 131 gegenüberliegenden Seite der Wellenscheiben 11, 13 ist eine wellenförmige Kontur ausgebildet, die sinusartig ausgebildet ist und zwei Maxima und zwei Minima aufweist, wobei die Kontur symmetrisch ausgebildet ist. Die mittlere Wellenscheibe 12 weist sowohl auf der Oberseite 121 als auch auf der Unterseite 122 eine Wellenkontur auf, wobei alle Wellenkonturen korrespondierend zueinander ausgebildet sind, so dass bei einer Ausrichtung der jeweiligen Minima zu den Minima der benachbarten Wellenscheibe 11, 12, 13 kein oder nahezu kein Zwischenraum zwischen den Wellenscheiben 11, 12, 13 vorhanden ist. Über die zwischen den Wellenscheiben 11, 12, 13 angeordneten Federscheiben 20 oder Federscheibenstapel 21 werden die Wellenscheiben 11, 12, 13 zueinander beabstandet gehalten. In der dargestellten Starrstellung sind jeweils Minima und Maxima zweier benachbarter, also in Axialrichtung hintereinander angeordneter Wellenscheiben 11, 12, 13 einander gegenüberliegend zugeordnet, so dass kein Federweg zwischen den Wellenscheiben 11, 12, 13 für die Federscheiben 20 oder Federscheibenpakete 21 zur Verfügung steht. Die von den endseitigen Wellenscheiben 11, 13 aufgebrachte Axialkraft wird über die Auflagepunkte auf der mittleren Wellenscheibe 12 weitergeleitet, ohne dass eine Federscheibe 20 elastisch verformt werden kann.

An dem radialen Umfang der mittleren Wellenscheibe 12 ist ein Formschlusselement 30 in Gestalt einer Ausnehmung ausgebildet, in die ein Stift eingeführt werden kann. Über dieses Formschlusselement 30 in Verbindung mit einem Eingriffselement ist es möglich, die mittlere Wellenscheibe 12 relativ zu den beiden anderen Wellenscheiben 11, 13 zu verdrehen. Dadurch ist es möglich, den möglichen Federweg, der durch die Federeinrichtung 1 ausgeübt werden kann, einzustellen. In der dargestellten Position ist keine Federung gegenüber die Federscheiben 20 möglich, da die Maxima der oberen Wellenscheibe 11 den oberen Maxima der mittleren Wellenscheibe 12 gegenüberliegen. Ebenso liegen die Maxima der unteren Wellenscheibe 13 den nach unten gerichteten Maxima oder Minima der Wellenscheibe 12 in der dargestellten Orientierung unmittelbar gegenüber, so dass sich zwischen den jeweils gegenüberliegenden Maxima kein freier Federweg befindet. Bevorzugt sind die endseitigen Wellenscheiben 11, 13 drehfest gelagert, entweder über radial wirkende Verriegelungen oder über an den flachen Stirnseiten 131, 111 angreifende Brems- oder Fixierelemente.

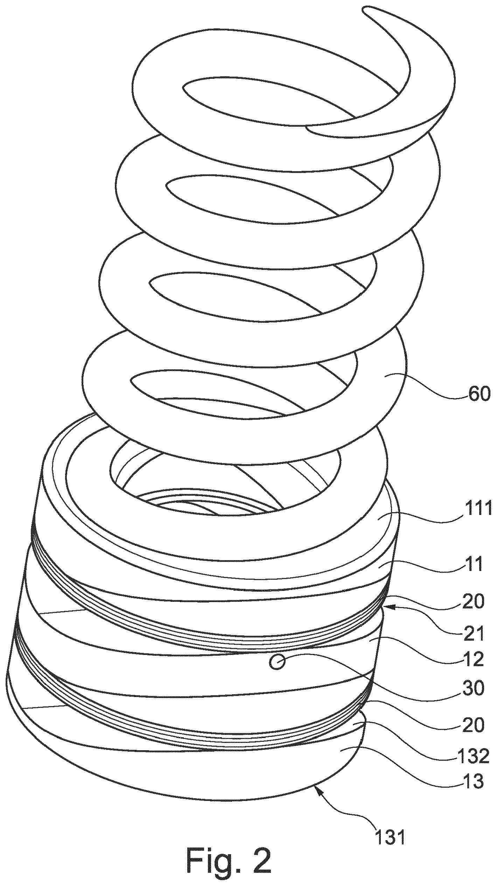

In der Darstellung gemäß Figur 2 ist bei gleicher Position der endseitigen Wellenscheiben 11, 13 die mittlere Wellenscheibe 12 um 90° um eine Achse entlang der Längserstreckung der Federeinrichtung 1 verdreht, was anhand der veränderten Position des Formschlusselementes 30 deutlich gemacht ist. Durch diese Drehung um 90° sind die jeweiligen Wellenkonturen aller Federscheiben 11, 12, 13 korrespondierend zueinander ausgerichtet, so dass die Konturverläufe der jeweils gegenüberliegenden Oberflächen einander entsprechen. Die Wellenscheiben 11, 12, 13 werden nur durch die dazwischen angeordneten Federscheiben 20 bzw. Federscheibenpakete 21 beabstandet zueinander gehalten. Wird nun eine Axialkraft in Richtung auf die Stirnseiten 111, 131 der endseitigen Wellenscheiben 11, 13 aufgebracht, um sie aufeinander zu zu verlagern, verformen sich die Federscheiben 20 in den Federscheibenpaketen 21 zwischen den jeweiligen Wellenscheiben 11, 12, 13 elastisch, während die Wellenscheiben 11, 12, 13 im Wesentlichen unverformt bleiben. Bei einer solchen Stellung der Wellenscheiben 11, 12, 13 zueinander ist über die Federeinrichtung 1 ein maximaler Federweg und eine maximale Energiespeichermenge erreichbar. Das Federelement 60 in Gestalt einer Schraubenfeder oder einer Wendelfeder dient als zusätzlicher mechanischer Energiespeicher und ist zu der Federeinrichtung 1 in Reihe geschaltet.

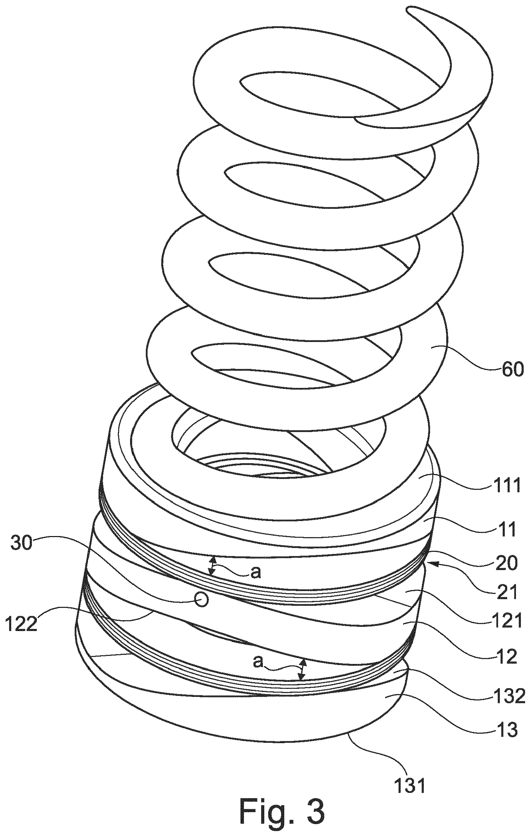

In der Figur 3 ist eine Zwischendarstellung zwischen den beiden Extremstellungen gemäß Figur 1 und 2 dargestellt, die mittlere Wellenscheibe 12 befindet sich ungefähr in der Mitte zwischen den beiden Extremstellungen gemäß Figuren 1 und 2. Der maximal mögliche Federweg ist der jeweils kürzeste Weg zwischen zwei Wellenscheiben 11, 12, 13, also der mögliche Weg zwischen eine Auflagestelle einer Federscheibe 20 oder einem Federscheibenpaket 21 und der benachbarten Wellenscheibenkontur in Axialrichtung. Der maximale Verlagerungsweg oder Federweg der Federeinrichtung besteht dann aus der Summe der freien Abstände a, wie sie in der Figur 3 gezeigt sind.

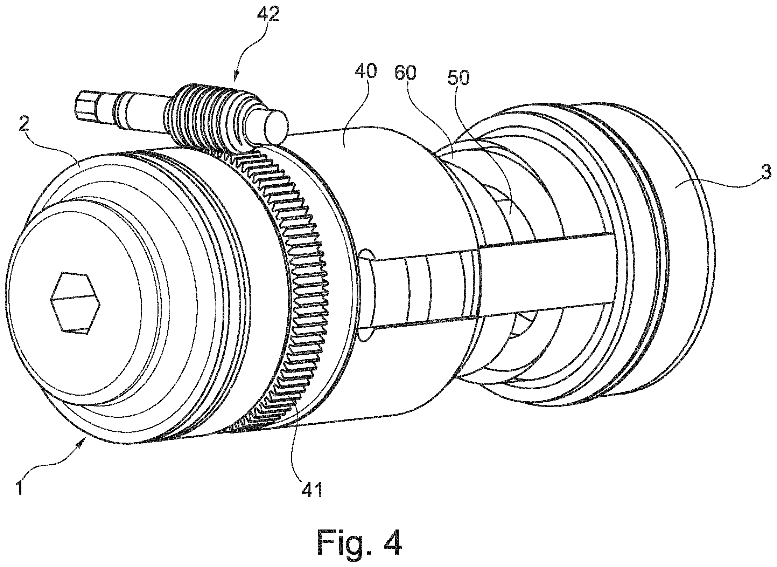

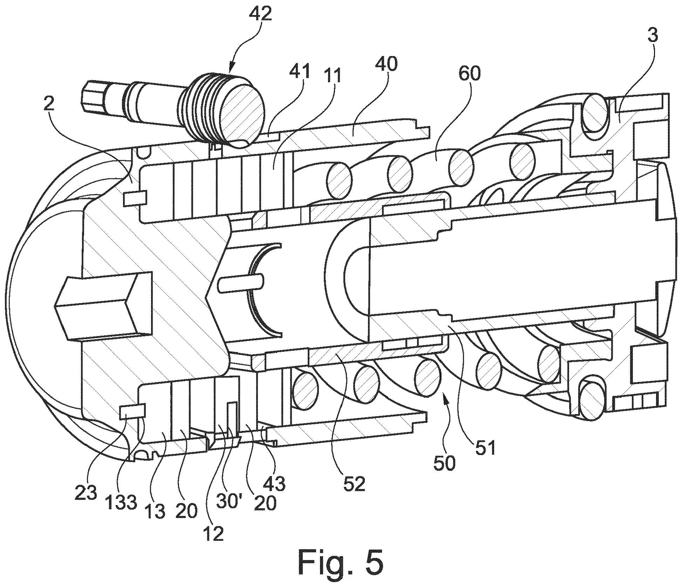

In der Figur 4 ist die Federeinrichtung 1 zusammen mit der Schraubenfeder oder Wendelfeder 60 in einem eingebauten Zustand zwischen zwei Endstücken 30 angeordnet dargestellt. Die Federeinrichtung 1 befindet sich innerhalb einer Verstelleinrichtung 40, die als Hülse ausgebildet ist und sowohl die Federscheiben 20 als auch die Wellenscheiben 11, 12, 13 umgibt. Innerhalb der Hülse 40 ist ein Schlitz 43 ausgebildet, in den ein Stift oder Formschlusselement 30` eingreift, wie es in der Figur 5 gezeigt ist. Dieser Stift 30' ist in das Formschlusselement 30 in der mittleren Wellenscheibe 12 eingeführt und ermöglicht über eine Verdrehung der Verstelleinrichtung 40 mit der Hülse eine Relativverdrehung der Federscheiben 11, 12, 13 relativ zu einander. Die beiden Endstücke 2, 3 halten die Wellenscheiben 11, 12, 13 und die Federscheiben 20 zusammen mit dem Federelement 60 zusammen. Zwischen den Endstücken 2, 3 ist eine Zentralführung 50 angeordnet, die durch Ausnehmungen innerhalb der Federscheiben 20 und Wellenscheiben 11, 12, 13 hindurchgeht und um die sich herum die Schraubenfeder 60 erstreckt. Das linke Endstück 2 weist an der Außenseite ein Gewinde auf, um es in einer Hydraulikeinheit zu befestigen, das gegenüberliegende Endstück 3 ist als Hydraulikkolben ausgebildet und kann relativ zu dem Endstück 2 verlagert werden, im dargestellten Ausführungsbeispiel auf das erste Endstück 2 zu.

An der Außenseite der Verstelleinrichtung 40 ist eine Verzahnung 41 ausgebildet, in die eine motorisch angetriebene Schnecke 42 eingreift. Über einen motorischen Antrieb, der nicht dargestellt ist, kann die Schnecke 42 in zwei Drehrichtungen angetrieben werden. Je nach Antriebsrichtung der Schnecke 42 wird die Verstelleinrichtung 40 in die eine oder andere Richtung um die Längserstreckung der Zentralführung 50 herum verdreht. Der Stift 30`, der in der Figur 5 dargestellt ist, wird formschlüssig über die hülsenartige Verstelleinrichtung 40 mitgenommen und verdreht dann die mittlere Wellenscheibe 12 relativ zu den beiden äußeren, drehfest gehaltenen Wellenscheiben 11, 13.

In der Figur 5 ist zu erkennen, dass die dem ersten Endstück 2 zugewandte Wellenscheibe 13 an der flachen Stirnseite 131 zwei Ausnehmungen 133 aufweist, in die zwei Stifte 23 hineinragen, die an dem Endstück 2 festgelegt sind. Dadurch wird eine Verdrehung der stirnseitigen Wellenscheibe 13 relativ zu der mittleren Wellenscheibe 12 ermöglicht und eine Verdrehung zu dem Endstück 2 verhindert, wenn die Verstelleinrichtung 40 relativ zu dem unbeweglichen Endstück 2 verdreht wird, wenn die Schnecke 42 angetrieben wird.

In der Figur 5 ist weiterhin zu erkennen, dass die Zentralführung 50 zweiteilig ausgebildet ist und zwei Rohrelemente 51, 52 aufweist, die ineinander schiebbar sind, so dass die beiden Endstücke 2, 3 zueinander verlagerbar sind. Die Zentralführung 50 ist somit teleskopierbar ausgebildet. In der Figur 5 ist ebenfalls zu erkennen, dass der Stift 30' in die radial nach außen zeigende Ausnehmung 30 der mittleren Wellenscheibe 12 eingreift und über den Schlitz 43 in der hülsenartigen Verstelleinrichtung 40 formschlüssig mitgenommen wird.

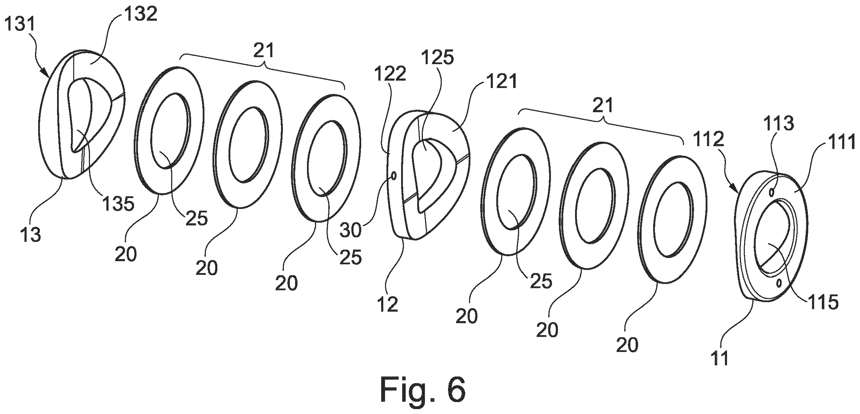

In der Figur 6 sind die Einzelteile einer Federeinrichtung 1 in Explosionsdarstellung gezeigt. Jeweils drei Federscheiben 20 sind zu einem Federscheibenstapel 21 zusammengefasst und zwischen zwei Wellenscheiben 11, 12, 13 angeordnet. Die endseitigen Wellenscheiben 11, 13 weisen an ihren ebenen Stirnseiten 111, 131 Ausnehmungen 113 bzw. 133 auf, in die Formschlusselemente, beispielsweise Stifte 23, eingreifen können, um eine Rotation relativ zu den Endstücken 2, 3 zu verhindern. Nur in der mittleren Wellenscheibe 12 ist die radial orientierte Bohrung 30 ausgebildet, die den Eingriff eines Formschlusselementes 30' ermöglicht, um eine Relativverdrehung der mittleren Wellenscheibe 12 zu den beiden äußeren, drehfest gelagerten Wellenscheiben 11, 13 zu ermöglichen. Die jeweiligen Oberflächenkonturen auf den einander zugewandten Stirnseiten 112, 132 der endseitigen Wellenscheiben 11, 13 entsprechen einander, ebenso entspricht die Kontur der mittleren Wellenscheiben 12 an ihren jeweiligen Oberflächen 123, 122 den Konturen der ihnen zugewandten Oberflächen 112, 132. Es sind jeweils auf der Oberfläche zwei Maxima und zwei Minima vorhanden, die mittleren Wellenscheibe 12 ist als beidseitig gewellte Scheibe ausgebildet, während die endseitigen Wellenscheiben 11, 13 nur an einer Stirnseite 112, 132 eine Wellenkontur aufweisen und somit als halbe Wellenscheiben angesehen werden können.

Sowohl in den Federscheiben 20 als auch in den Wellenscheiben 11, 12, 13 sind Zentralausnehmungen 115, 125, 135, 25 ausgebildet, die im dargestellten Ausführungsbeispiel rund ausgebildet sind. Über die runde Ausgestaltung zumindest der mittleren Wellenscheibe 12 am Außenumfang ebenso wie im Innenumfang der Ausnehmung 125 ist es möglich, eine Rotation um die Zentralausnehmung 50 herum auszuführen.

Statt der stirnseitigen Festlegung über Bohrungen 113, 133 an den endseitigen Wellenscheiben 11, 13 kann auch eine radial gerichtete Festlegung, beispielsweise an der Zentralausnehmung 50, stattfinden, beispielsweise durch eine unrunde Ausgestaltung mit einer Abflachung in dem rohrförmigen Element 52 und eine korrespondierende unrunde Zentralausnehmung 115 der jeweiligen Wellenscheibe 11, 13.

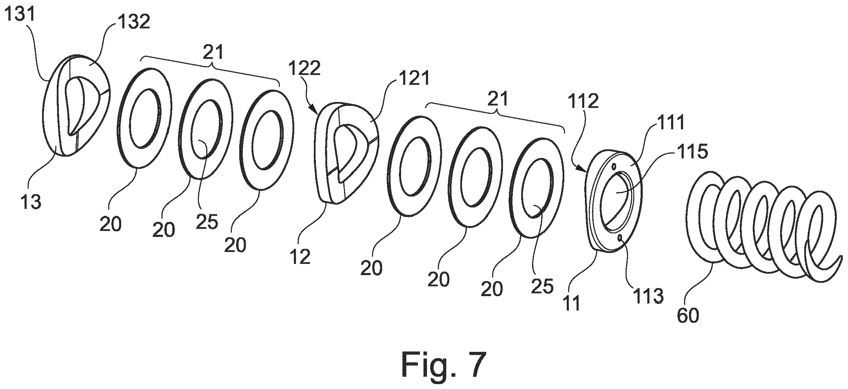

In der Figur 7 ist die Federeinrichtung gemäß Figur 6 mit dem in Axialrichtung vorgeschalteten Federelement 60 in Gestalt der Schraubenfeder gezeigt.

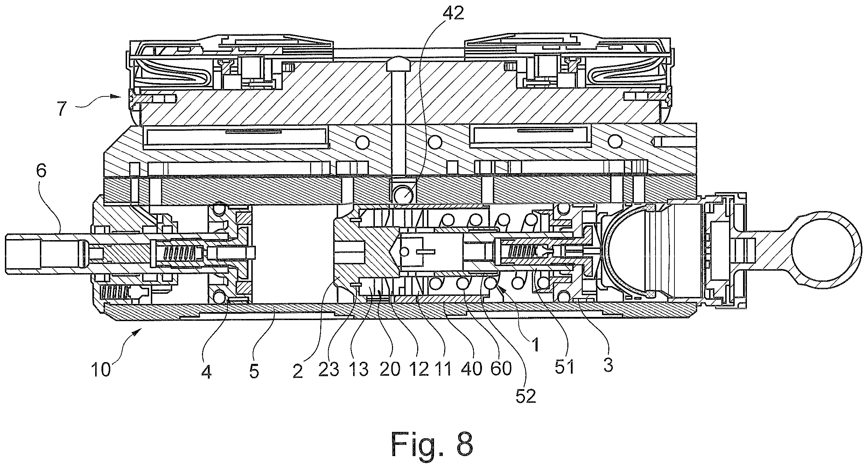

Figur 8 zeigt einen Hydraulikaktuator 10 in Gestalt eines Hydraulikdämpfers mit einem Gehäuse 5, in dem ein Hydraulikkolben 4 über eine Kolbenstange 6 axial verschieblich gelagert ist. Innerhalb des Gehäuses 5 ist das erste Endstück 2 über ein Außengewinde eingeschraubt, das zweite Endstück 3 der Federeinrichtung 1 ist als beweglicher Kolben mit einem am Außenumfang angeordneten Dichtelement ausgebildet. Der Hydraulikaktuator 10 weist eine elektronische Steuerungseinrichtung 7 auf, über die Ventile geöffnet und geschlossen werden können. Ebenso wird über die Steuereinrichtung 7 der nicht dargestellte Antrieb für die Schnecke 42 aktiviert oder deaktiviert, die in die Außenverzahnung 41 der Verstelleinrichtung 40 eingreift. Die linke, endseitige Wellenscheibe 13 ist über die Stifte 23 drehfest an dem Endstück 2 gelagert. In Axialrichtung schließt sich ein erster Federstapel 21, die mittlere Wellenscheibe 12, ein zweiter Federstapel 21 und die zweite, endseitige Wellenscheibe 11 an. Wird die Kolbenstange 6 in Richtung auf das zweite Endstück 2 verlagert, wird Hydraulikfluid über Hydraulikkanäle nach rechts in Richtung auf das zweite Endstück 3 gedrückt. Der Hydraulikdruck komprimiert das Federelement 60, das sich auf der ebenen Seitenfläche 111 abstützt und beispielsweise über eine formschlüssige Kopplung über die Wendelfeder 60 ebenfalls drehfest in der hülsenartigen Verstelleinrichtung 40 gelagert ist. Durch das Federelement 60 wird eine Axialkraft ausgeübt, die in Richtung auf die zweite endseitige Wellenscheibe 13 wirkt. Bei einer Stellung der Wellenscheiben 11, 12, 13 zueinander gemäß Figur 1 würden die Federscheiben 20 zwischen den Wellenscheiben 11, 12, 13 keinen Federweg zur Verfügung haben, so dass die einzige Energiespeichereinrichtung die Schraubenfeder 60 sein würde. Wird eine andere Energiespeichermenge benötigt, wird der nicht dargestellte Antrieb aktiviert, die Schnecke 42 greift in die Verzahnung 41 ein, verdreht die Verstelleinrichtung 40 beispielsweise um 90°, bis die Stellung gemäß Figur 2 erreicht wird. Dann wird ein zusätzlicher Federweg und eine zusätzliche Federkraft bzw. Energiespeichermenge zur Verfügung gestellt.

Zwischen den beiden Extrempositionen gemäß Figur 1 und 2 kann jede Position eingenommen werden, so dass jeder Federweg zwischen Null und dem maximalen Federweg einstellbar ist.

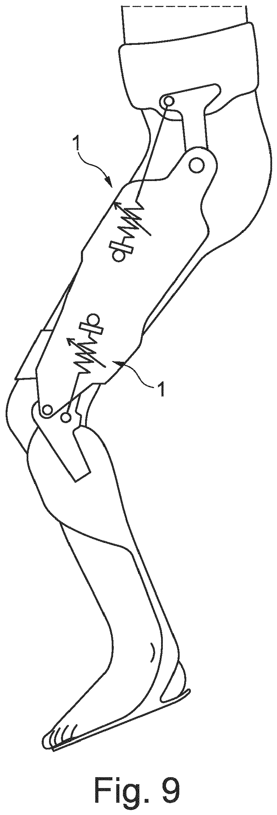

Mögliche Anordnungen der Federeinrichtung 1 oder auch des Hydraulikaktuators gemäß Figur 8 sind in der Figur 9 gezeigt, bei der die schematisch angedeuteten Federeinrichtungen 1 an der Außenseite einer Orthese angeordnet sind. Die verstellbare Federeinrichtung 1 ist zwischen einem Hüftgurt oder Hüftschale und einem Oberschenkelrahmen sowie zwischen einem Oberschenkelrahmen und einem Unterschenkelteil einer dreiteiligen Orthese ausgebildet. Die dargestellte Orthese weist ein Unterschenkelteil mit einem Fußteil auf, wobei das Fußteil nicht gelenkig mit dem Unterschenkelteil verbunden ist. Grundsätzlich ist es auch möglich, eine Federeinrichtung 1 zwischen einer Fußplatte und einem Unterschenkelteil, sofern diese gelenkig miteinander verbunden sind, anzuordnen.

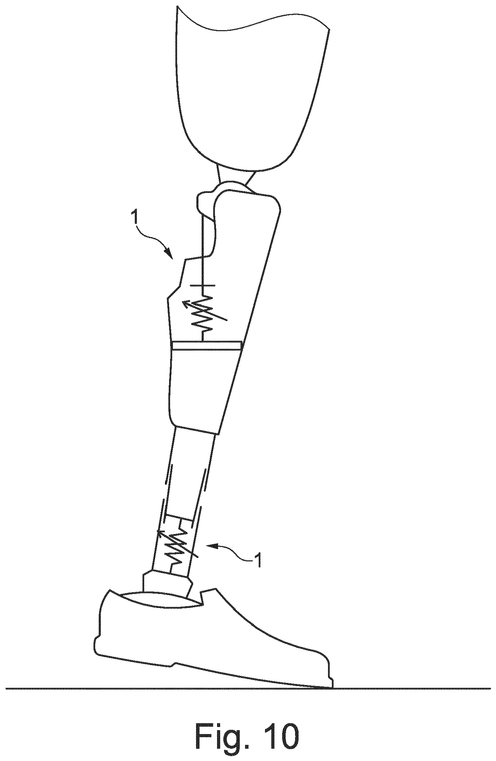

Eine Variante der Erfindung sieht die Anordnung der Federeinrichtung 1 in einer Prothese vor, wie dies in der Figur 10 dargestellt ist. Zwei Federeinrichtungen 1 oder Hydraulikaktuatoren können zwischen einem Prothesenfuß und einem Unterschenkelteil oder zwischen einem Unterschenkelteil und einem Oberschenkelteil in einem Prothesenkniegelenk angeordnet sein. Die Federeinrichtung 1 zwischen dem Prothesenfuß und dem Unterschenkelrohr lässt eine einstellbare Energiespeicherung bei einer Axialbelastung in Richtung auf das Prothesenkniegelenk entlang dem Unterschenkelrohr zu. Die zwischen dem Unterschenkelteil und dem Prothesenkniegelenk angeordnete Federeinrichtung 1 dient beispielsweise zur Speicherung von Bewegungsenergien während der Flexion und zur Freigabe während der Extension.

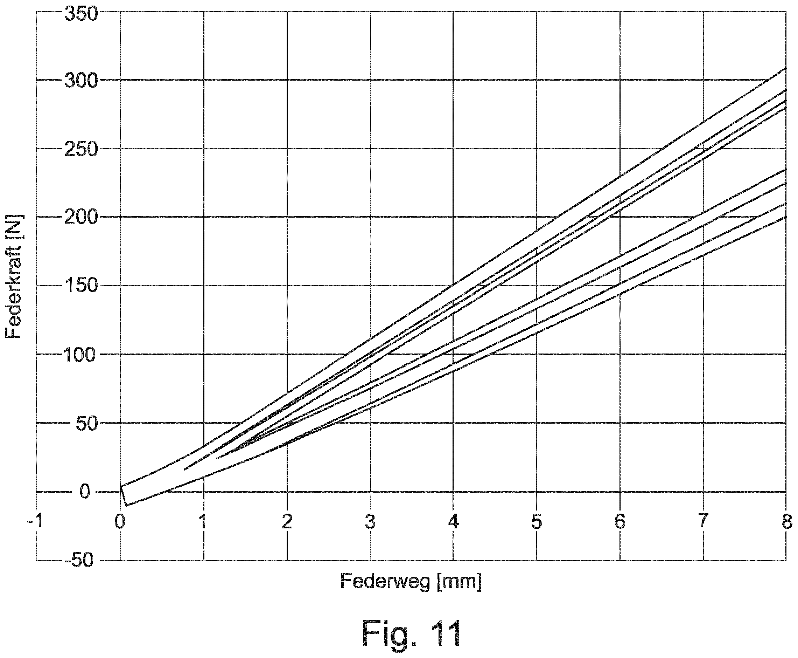

Ein Diagramm des Federweges A über die Federkraft N ist in der Figur 11 gezeigt, auf dem zu erkennen ist, dass durch die Verstellung der Wellenscheiben 11, 12, 13 zueinander die aufzunehmende Federkraft ebenso wie der maximale Federweg verstellt werden können.

Die Steifigkeit der Federeinrichtung 1 kann verändert werden, ohne dass sich die Länge der Federeinrichtung 1 ändert. Ebenso kann die zu speichernde und damit auch die abzugebende Energiemenge stufenlos verändert werden. Neben der motorischen Verstellung über die Schnecke 42 kann auch eine manuelle Verstellung über einen Hebel oder einen anderen Zugang zu der verstellbaren Wellenscheibe erfolgen. Es können durch Hintereinanderschaltung mehrerer Wellenscheiben mit beidseitigen Wellenkontur und zwischengelegten Federscheiben einfach Veränderungen in dem maximalen Federweg und der zu speichernden Energiemenge vorgenommen werden. Die drehbar gelagerten Wellenscheiben sind individuell einstellbar, um jede Kombination von aktivierten, teilweise aktivierten und deaktivierten federscheiben 20 oder Federstapel 21 einstellen zu können.

## Patentansprüche

1. Federeinrichtung mit zumindest zwei Wellenscheiben (11, 12, 13) aus einem formstabilen Material, die starr ausgeführt sind und zumindest eine gewellte Oberfläche aufweisen, mit zumindest einer zwischen den Wellenscheiben (11, 12, 13) angeordneten Federscheibe (20), wobei die Wellenscheiben (11, 12, 13) zueinander verdrehbar gelagert sind, **dadurch gekennzeichnet, dass** zumindest eine Wellenscheibe (11, 12, 13) mit einer motorisch angetriebenen Verstelleinrichtung (40) gekoppelt ist.

2. Federeinrichtung nach Anspruch 1, **dadurch gekennzeichnet, dass** mehrere Federscheiben (20) als Federscheibenstapel (21) zwischen den Wellenscheiben (11, 12, 13) angeordnet sind.

3. Federeinrichtung nach Anspruch 1 oder 2, **dadurch gekennzeichnet, dass** eine endseitig angeordnete Wellenscheibe (11, 13) auf der der Federscheibe (20) abgewandten Seite (111, 131) eben ausgebildet ist.

4. Federeinrichtung nach einem der voranstehenden Ansprüche, **dadurch gekennzeichnet, dass** zwischen zwei Federscheiben (20) eine Wellenscheibe (12) angeordnet ist, die auf beiden den Federscheiben (20) zugewandten Seiten (121, 122) wellig ausgebildet ist.

5. Federeinrichtung nach einem der voranstehenden Ansprüche, **dadurch gekennzeichnet, dass** die Wellenscheiben (11, 12, 13) und die Federscheibe (20) jeweils eine zentrale Ausnehmung (115, 125, 135; 25) aufweisen und auf einer Zentralführung (50) gelagert sind.

6. Federeinrichtung nach einem der voranstehenden Ansprüche, **dadurch gekennzeichnet, dass** die zumindest eine Wellenscheibe (11, 12, 13) über ein Formschlusselement (30, 30'), kraftschlüssig oder stoffschlüssig mit der Verstelleinrichtung (40) gekoppelt ist.

7. Federeinrichtung nach Anspruch 6, **dadurch gekennzeichnet, dass** das Formschlusselement (30) als Ausnehmung, Vorsprung, Stift, Absatz, Unrundheit oder Verzahnung ausgebildet ist.

8. Federeinrichtung nach Anspruch 6, **dadurch gekennzeichnet, dass** die Verstelleinrichtung (40) die Wellenscheibe (11, 12, 13) zumindest teilweise umgibt oder innerhalb der Wellenscheibe (11, 12, 13) angeordnet ist.

9. Federeinrichtung nach Anspruch 1, **dadurch gekennzeichnet, dass** die Verstelleinrichtung (40) eine Verzahnung (41) aufweist, die mit einem angetriebenen Zahnrad oder einer angetriebenen Schnecke (42) gekoppelt ist.

10. Federeinrichtung nach einem der voranstehenden Ansprüche, **dadurch gekennzeichnet, dass** alle Wellenscheiben (11, 12, 13) die gleiche Anzahl an Wellen und die gleichen Wellenform aufweisen.

11. Federeinrichtung nach einem der voranstehenden Ansprüche, **dadurch gekennzeichnet, dass** sie zwischen zwei zueinander verlagerbaren Endstücken (2, 3) angeordnet ist.

12. Federeinrichtung nach Anspruch 5 oder 11, **dadurch gekennzeichnet, dass** eine Wellenscheibe (11, 13) drehfest an der Zentralführung (50) und/oder an einem der Endstücke (2, 3) gelagert ist.

13. Federeinrichtung nach Anspruch 11 oder 12, **dadurch gekennzeichnet, dass** die Endstücke (2, 3) teleskopierbar aneinander festgelegt sind.

14. Federeinrichtung nach einem der Ansprüche 11 bis 1333 in Verbindung mit Anspruch 5, **dadurch gekennzeichnet, dass** die Endstücke (2, 3) über die Zentralführung (50) miteinander verbunden sind.

15. Federeinrichtung nach einem der Ansprüche 11 bis 14, **dadurch gekennzeichnet, dass** zwischen einem Endstück (2, 3) und zumindest einer Wellenscheibe (11, 13) ein Federelement (60) angeordnet ist.

16. Federeinrichtung nach einem der voranstehenden Ansprüche, **dadurch gekennzeichnet, dass** die Wellenscheiben (11, 12, 13) eine sinusartige, dreieckige, trapezartige oder rechteckige Form mit zumindest zwei Maxima und zwei Minima aufweisen.

17. Hydraulikaktuator (10) mit einer Federeinrichtung (1) nach einem der voranstehenden Ansprüche.

## Claims

1. A spring device having at least two wave washers (11, 12, 13) made of a dimensionally stable material, which are rigid and have at least one undulated surface, having at least one spring washer (20) disposed between the wave washers (11, 12, 13), wherein the wave washers (11, 12, 13) are mounted so as to be capable of being rotated relative to one another, **characterized in that** at least one wave washer (11, 12, 13) is coupled to a motor-driven adjustment device (40).

2. The spring device as claimed in claim 1, **characterized in that** a plurality of spring washers (20) as a spring washer stack (21) are disposed between the wave washers (11, 12, 13).

3. The spring device as claimed in claim 1 or 2, **characterized in that** a wave washer (11, 13) arranged at the end is configured to be flat on the side (111, 131) facing away from the spring washer (20).

4. The spring device as claimed in one of the preceding claims, **characterized in that** a wave washer (12) which is configured so as to be undulated on both sides (121, 122) that face the spring washers (20) is disposed between two spring washers (20).

5. The spring device as claimed in one of the preceding claims, **characterized in that** the wave washers (11, 12, 13) and the spring washer (20) have in each case a central clearance (115, 125, 135; 25) and are mounted on a central guide (50).

6. The spring device as claimed in one of the preceding claims, **characterized in that** at least one wave washer (11, 12, 13) is coupled to the adjustment device (40) by way of a form-fit element (30, 30'), frictionally or materially.

7. The spring device as claimed in claim 6, **characterized in that** the form-fit element (30) is configured as a clearance, a protrusion, a pin, a shoulder, an eccentric, or a toothing.

8. The spring device as claimed in claim 6, **characterized in that** the adjustment device (40) at least partially surrounds the wave washer (11, 12, 13) or is disposed within the wave washer (11, 12, 13).

9. The spring device as claimed in claim 1, **characterized in that** the adjustment device (40) has a toothing (41) which is coupled to a driven gear wheel or a driven worm (42).

10. The spring device as claimed in one of the preceding claims, **characterized in that** all wave washers (11, 12, 13) have the same number of undulations and the same wave shape.

11. The spring device as claimed in one of the preceding claims, **characterized in that** said spring device is disposed between two mutually displaceable end pieces (2, 3).

12. The spring device as claimed in claim 5 or 11, **characterized in that** a wave washer (11, 13) is mounted in a rotationally fixed manner on the central guide (50) and/or on one of the end pieces (2, 3).

13. The spring device as claimed in claim 11 or 12, **characterized in that** the end pieces (2, 3) are mutually established in a telescopic manner.

14. The spring device as claimed in one of claims 11 to 13, in conjunction with claim 5, **characterized in that** the end pieces (2, 3) are connected to one another by way of the central guide (50).

15. The spring device as claimed in one of claims 11 to 14, **characterized in that** a spring element (60) is disposed between an end piece (2, 3) and at least one wave washer (11, 13).

16. The spring device as claimed in one of the preceding claims, **characterized in that** the wave washers (11, 12, 13) have a sinusoidal, triangular, trapezoidal, or rectangular shape having at least two maxima and two minima.

17. A hydraulic actuator (10) having a spring device (1) as claimed in one of the preceding claims.

## Revendications

1. Dispositif à ressort comprenant au moins deux rondelles ondulées (11, 12, 13) en un matériau solide en forme, qui sont réalisées de manière rigide et présentent au moins une surface ondulée, comprenant au moins une rondelle élastique (20) disposée entre les rondelles ondulées (11, 12, 13), les rondelles ondulées (11, 12, 13) étant logées de manière à pouvoir tourner l'une par rapport à l'autre,
**caractérisé en ce qu'**au moins une rondelle ondulée (11, 12, 13) est couplée à un dispositif de réglage (40) entraîné par voie motrice.

2. Dispositif à ressort selon la revendication 1,
**caractérisé en ce que** plusieurs rondelles élastiques (20) sont disposées sous forme d'empilement de rondelles élastiques (21) entre les rondelles ondulées (11, 12, 13).

3. Dispositif à ressort selon la revendication 1 ou 2,
**caractérisé en ce qu'**une rondelle ondulée (11, 13) disposée du côté extrémité est réalisée plane sur la face (111, 131) détournée de la rondelle élastique (20).

4. Dispositif à ressort selon l'une des revendications précédentes,
**caractérisé en ce qu'**une rondelle ondulée (12) est disposée entre deux rondelles élastiques (20), qui est réalisée de façon ondulée sur les deux faces (121, 122) tournées vers les rondelles élastiques (20).

5. Dispositif à ressort selon l'une des revendications précédentes,
**caractérisé en ce que** les rondelles ondulées (11, 12, 13) et la rondelle élastique (20) présentent chacune un évidement central (115, 125, 135 ; 25) et sont logées sur un guidage central (50).

6. Dispositif à ressort selon l'une des revendications précédentes,
**caractérisé en ce que** ladite au moins une rondelle ondulée (11, 12, 13) est couplée par coopération de force ou de matière au dispositif de réglage (40) par l'intermédiaire d'un élément de coopération de forme (30, 30').

7. Dispositif à ressort selon la revendication 6,
**caractérisé en ce que** l'élément de coopération de forme (30) est réalisé sous forme d'évidement, de saillie, de broche, d'épaulement, d'ovalisation ou de denture.

8. Dispositif à ressort selon la revendication 6,
**caractérisé en ce que** le dispositif de réglage (40) entoure au moins partiellement la rondelle ondulée (11, 12, 13) ou est disposé à l'intérieur de la rondelle ondulée (11, 12, 13).

9. Dispositif à ressort selon la revendication 1,
**caractérisé en ce que** le dispositif de réglage (40) présente une denture (41) qui est couplée à une roue dentée entraînée ou à une vis sans fin entraînée (42).

10. Dispositif à ressort selon l'une des revendications précédentes,
**caractérisé en ce que** toutes les rondelles ondulées (11, 12, 13) présentent le même nombre d'ondulations et la même forme d'ondulation.

11. Dispositif à ressort selon l'une des revendications précédentes,
**caractérisé en ce qu'**il est disposé entre deux pièces d'extrémité (2, 3) pouvant être déplacées l'une par rapport à l'autre.

12. Dispositif à ressort selon la revendication 5 ou 11,
**caractérisé en ce qu'**une rondelle ondulée (11, 13) est montée solidairement en rotation sur le guidage central (50) et/ou sur l'une des pièces d'extrémité (2, 3).

13. Dispositif à ressort selon la revendication 11 ou 12,
**caractérisé en ce que** les pièces d'extrémité (2, 3) sont fixées l'une à l'autre de manière télescopique.

14. Dispositif à ressort selon l'une des revendications 11 à 13 en combinaison avec la revendication 5,
**caractérisé en ce que** les pièces d'extrémité (2, 3) sont reliées entre elles par le guidage central (50).

15. Dispositif à ressort selon l'une des revendications 11 à 14,
**caractérisé en ce qu'**un élément ressort (60) est disposé entre une pièce d'extrémité (2, 3) et au moins une rondelle ondulée (11, 13).

16. Dispositif à ressort selon l'une des revendications précédentes,
**caractérisé en ce que** les rondelles ondulées (11, 12, 13) présentent une forme sinusoïdale, triangulaire, trapézoïdale ou rectangulaire avec au moins deux maxima et deux minima.

17. Actionneur hydraulique (10) comprenant un dispositif à ressort (1) selon l'une des revendications précédentes.
